# EUROPÄISCHE PATENTSCHRIFT

(11) **EP 0 347 689 B1**
(45) Veröffentlichungstag und Bekanntmachung des Hinweises auf die Patenterteilung: **22.09.1993**
(21) Anmeldenummer: 89110557.9
(22) Anmeldetag: 10.06.1989
(51) Int. Cl.: C07D 231/16

(54) **Verfahren zur Herstellung von N-Hydroxypyrazolen**
Process for the preparation of N-hydroxypyrazoles
Procédé de préparation de pyrazoles N-hydroxyliques

(30) Priorität: 18.06.1988 DE 3820739
(43) Veröffentlichungstag der Anmeldung: 27.12.1989
(73) Patentinhaber: BASF Aktiengesellschaft, 67063 Ludwigshafen (DE)
(72) Erfinder: Baus, Ulf, Dr., D-6915 Dossenheim (DE); Reuther, Wolfgang, Dr., D-6900 Heidelberg (DE); Hahn, Erwin, Dr., D-6900 Heidelberg (DE)

(56) Entgegenhaltungen:
- EP-A- 0 215 380
- DE-A- 3 031 385
- DE-A- 3 409 317
- METHODEN DER ORGANISCHEN CHEMIE, Houben-Weyl, Band X/1, 1971, Teil 1, Nr. B34100, Seiten 1135-1137, Georg Thieme Verlag, Stuttgart, DE; "Stickstoff-Verbindungen I"
- THE JOURNAL OF ORGANIC CHEMISTRY, Band 34, Nr. 1, Januar 1969, Seiten 187-194, Easton, US; J.P. FREEMAN et al.: "The nitrosation of alpha,beta-unsaturated oximes. IV. The synthesis and structure of 3,4-diazacyclopentadienone derivatives"

## Beschreibung

Die Erfindung betrifft die Herstellung von N-Hydroxypyrazol und seinen Derivaten aus den entsprechenden Pyrazolen.

N-Hydroxypyrazol und seine Derivate sind wichtige Zwischenprodukte für die Herstellung von Substanzen mit breitem biologischen Wirkungsspektrum. Beispielsweise wird in DE 3 409 317 die Herstellung vor wertvollen Nitrifikationsinhibitoren für Ammoniumstickstoff aus N-Hydroxypyrazolen beschrieben. Weiterhin wird in DE 35 32 880 die Herstellung von 1,4-disubstituierten Derivaten des N-Hydroxypyrazols als Verbindungen mit selektiver Wirkung auf Histamin-H₂-Rezeptoren beschrieben. Es hat daher nicht an Versuchen gefehlt ein vorteilhaftes Verfahren zur Herstellung von N-Hydroxypyrazolen zu finden.

Gemäß der DE-OS 3 031 385 wird N-Hydroxypyrazol durch eine mehrstufige Synthese aus den Bausteinen Azodicarbonsäureestern, Cyclopentadien, Nitriloxid und einem Peroxid hergestellt. Nachteilig an diesem Verfahren ist die mehrstufige Reaktionsführung, die geringe Ausbeute sowie die Tatsache, daß das N-Hydroxypyrazol im Gemisch mit Isoxazolen anfällt.

Aus DE-OS 3 205 456 ist ein Verfahren zur Herstellung von halogenierten N-Hydroxypyrazolen bekannt, mit dessen Hilfe die gemäß dem Verfahren von DE-OS 3 031 385 hergestellten N-Hydroxypyrazole halogeniert werden können.

Kernsubstituierte N-Hydroxypyrazole können beispielsweise gemäß J. Org. Chem. 34 (1969), Seiten 187-94 durch Nitrosierung entsprechend substituierter α,β-ungesättigter Oxime und anschließende Reduktion der erhaltenen 3,4-Diazacyclopentacienondioxide hergestellt werden. Nachteilig an diesem Verfahren sind die mehrstufige Verfahrensführung sowie die Tatsache, daß nur kernsubstituierte N-Hydroxypyrazole hergestellt werden können.

Es war daher die Aufgabe der Erfindung, ein einfaches und wirtschaftliches Verfahren zu finden, das die Herstellung sowohl von N-Hydroxypyrazol selbst als auch von kernsubstituierten N-Hydroxypyrazolen ermöglicht.

Es wurde nun überraschenderweise gefunden, daß man N-Hydroxypyrazol selbst sowie kernsubstituierte N-Hydroxypyrazole auf einfache Weise erhält, wenn man die entsprechencen Pyrazole mit einer Peroxocarbonsäure umsetzt.

Es war zwar bekannt, daß sich bei der Umsetzung von primären oder sekundären aliphatischen Aminen mit Wasserstoffperoxid oder seinen Acylderivaten wie Dibenzoylperoxid in Verbindung mit Katalysatoren und anschließende Verseifung der dabei gebildeten O-Benzoyl-N-hydroxylamine Hydroxylamine herstellen lassen (vgl. Houben-Weyl, Methoden der organischen Chemie, Bd. 10/1, Thieme-Verlag, 1971, Seiten 1135-1137), jedoch wird in loc. cit. diese Reaktion ausdrücklich auf aliphatische Amine beschränkt.

Gegenstand der Erfindung ist dementsprechend ein Verfahren zur Herstellung von N-Hydroxypyrazolen der allgemeinen Formel I 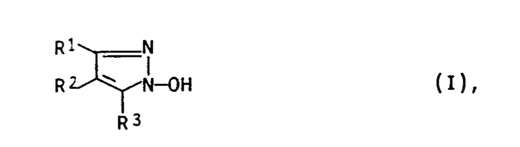
in der
- R¹, R² und R³: gleich oder verschieden sein können und Wasserstoff oder Halogen bedeuten können,
das dadurch gekennzeichnet ist, daß man
Pyrazole der allgemeinen Formel II 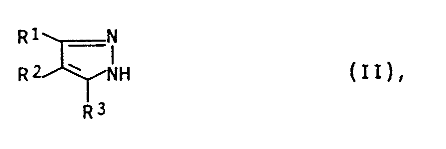
in der
die Reste R¹, R² und R³ die in Formel I angegebene Bedeutung haben, in Gegenwart von 1 bis 15 Mol eines Alkali- oder Erdalkalihydroxids oder -carbonats mit einer aliphatischen oder aromatischen Peroxocarbonsäure in einem stöchiometrischen Verhältnis von Pyrazol II und Peroxocarbonsäure = 1:1 bis 1:2 so umsetzt, daß die Reaktionstemperatur zwischen -5°C und 60°C liegt.

Besonders gute Ausbeuten werden bei dieser Umsetzung erzielt, wenn man sie in Gegenwart von 1,5 bis 9 Mol eines Alkali- oder Erdalkalihydroxids, bezogen auf das Pyrazol, durchführt. Dabei kann es von Vorteil sein, wenn man die Pyrazole der Formel II vor der Umsetzung mit der Peroxocarbonsäure in an sich bekannter Weise mit einem Alkalihydroxid, Alkalihydrid oder Alkalicarbonat in ihre Metallsalze der allgemeinen Formel III 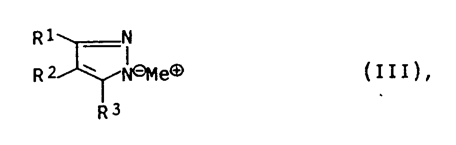
in der
die Reste R¹, R² und R³ die in Formel I genannte Bedeutung haben, und
Me^{⊕} ein Kation der Alkalimetalle bedeutet,
überführt.

Als Pyrazole der allgemeinen Formel II kommen in Betracht: Pyrazol, 3-Chlor-pyrazol, 4-Chlor-pyrazol, 3-Brom-pyrazol, 4-Brom-pyrazol, 3-Jod-pyrazol, 4-Jod-pyrazol, 3,4-Dichlor-pyrazol, 3,4,5-Trichlorpyrazol, 3,4-Dibrom-pyrazol, 3,4,5-Tribrom-pyrazol, 3,4-Dijod-pyrazol und 3,4,5-Trijodpyrazol.

Die Überführung der Pyrazole in ihre Metallsalze der allgemeinen Formel III erfolgt in an sich bekannter Weise durch Umsetzen der Pyrazole in Wasser oder einem inerten Lösungsmittel mit einem Alkalihydroxid, Alkalihydrid oder Alkalicarbonat bei Temperaturen von 0 bis 60°C.

Als Peroxocarbonsäure eignen sich sowohl aromatische Peroxocarbonsäuren, wie m-Chlorperbenzoesäure, Perbenzoesäure und Monoperphthalsäure als auch aliphatische, wie Peressigsäure, Perpropionsäure, Trifluorperessigsäure und Monoperbernsteinsäure. Besonders vorteilhaft ist die Verwendung von Monoperphthalsäure.

Das stöchiometrische Verhältnis von Pyrazol II und Peroxosäure beträgt im allgemeinen 1:1 bis 1:2.

Die Umsetzung wird im allgemeinen in einem Lösungsmittel durchgeführt.

Als Lösungsmittel für diese Umsetzung seien aliphatische Ether, wie Diethylether, Diisopropylether, Diethylenglykoldimethylether; cyclische Ether, wie Tetrahydrofuran (THF) und Dioxan sowie aromatische Kohlenwasserstoffe, wie Toluol und Halogenkohlenwasserstoffe, wie Methylenchlorid, Chloroform und Chlorbenzol genannt.

Besonders vorteilhaft gestaltet sich in vielen Fällen die Verwendung von Wasser als Lösungsmittel. Bei Einsatz von hydrolyseempfindlichen Peroxocarbonsäuren kann mit Vorteil in einem 2-Phasensystem aus Wasser und einem mit Wasser nicht oder nur schlecht mischbaren Lösungsmittel gearbeitet werden. Hierbei kommen als Lösungsmittel im wesentlichen aliphatische, aromatische und chlorierte Kohlenwasserstoffe in Betracht. Gennant seien insbesondere Cyclohexan, Toluol oder Methylenchlorid.

Beim Arbeiten in einem geeigneten 2-Phasensystem empfiehlt es sich in Gegenwart eines geeigneten Phasentransferkatalysators zu arbeiten.

Für das erfindungsgemäße Verfahren lassen sich zahlreiche Phasentransfer-Katalysatoren (allg. Übersicht vgl. V. Dehmlow, Angew. Chemie, 89 (1977), Seite 521 bis 533) verwenden. Besonders bevorzugte Phasentransferkatalysatoren (PTK) sind:
1. Tetraalkylammoniumsalze der allgemeinen Formel IV 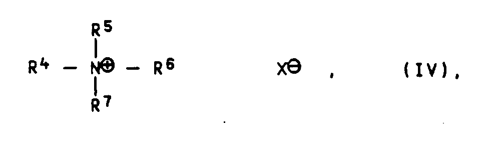 in der
   R⁴, R⁵, R⁶, und R⁷ untereinander gleich oder verschieden sein können und für Alkylreste mit 1 bis 22 Kohlenstoffatomen oder für funktionelle Gruppen, wie Hydroxyl-, Carbonamid- oder Ethergruppen enthaltende Alkylreste mit bis zu 25 Kohlenstoffatomen wie Methyl-, Ethyl-, (Iso)propyl-, Butyl-, Octyl-, Dodecyl-, C₁₆H₃₃-, Hydroxy(iso)-propyl- oder 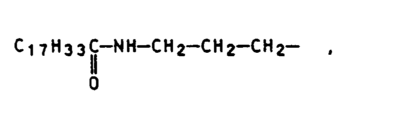 sowie für Phenylreste oder phenylsubstituierte Alkylreste (wie. z.B. Benzyl-) mit bis zu 20 Kohlenstoffatomen stehen können,
   und X^{⊖} für ein Anion Säure wie J⁻, Cl⁻, Br⁻, (HSO₄)⁻, (CN)⁻, (BF₄)⁻ oder OH⁰ steht; insbesondere das sehr preiswerte Trimethylbenzylammoniumchlorid, das in Form seiner 50 %igen wäßrigen Lösung eingesetzt werden kann, sowie Tricaprylmethylammoniumchlorid; und
2. Tetraalkylphosphoniumsalze der allgemeinen Formel V 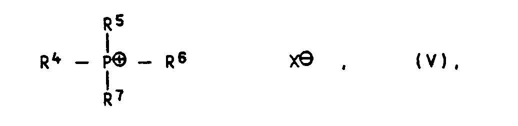 worin R⁴, R⁵, R⁶, R⁷ sowie X^{⊖} die für die Formel (IV) angegebene Bedeutung haben können, insbesondere das Tri-n-octyl-methylphosphoniumiodid.

Weiterhin geeignet sind auch Gemische der oben genannten PTK sowie Trägerfixierte PTK.

Die PTK werden für das erfindungsgemäße Verfahren in Mengen von 0,1 bis 1, vorzugsweise 0,3 bis 0,5 Mol pro Mol Pyrazol eingesetzt.

Die Umsetzung setzt - je nach Reaktivität der verwendeten Peroxocarbonsäure schon bei Temperaturen unterhalb 0°C ein, in den meisten Fällen jedoch erst bei Temperaturen von ca. 20°C.

Die Umsetzung gelingt besonders gut in Gegenwart größerer Mengen einer Base wie einem Alkali- oder Erdalkalihydroxid oder -carbonat. Bevorzugt setzt man dem Reaktionsgemisch noch vor Zugabe der Peroxocarbonsäure mindestens 1 Äquivalent der Base zu. Man kann aber auch anstelle der Peroxocarbonsäure eines ihrer Metallsalze, insbesondere ein Alkalimetallsalz oder Erdalkalimetallsalz der Peroxocarbonsäure verwenden.

Weiterhin besteht die Möglickeit, die Peroxocarbonsäure vor der Umsetzung im Reaktionsgemisch in an sich bekannter Weise herzustellen. Hierzu setzt man Wasserstoffperoxid beispielsweise mit einem Carbonsäurehalogenid, insbesondere einem Carbonsäurechlorid, oder einem Carbonsäureanhydrid um. Auch hierbei ist bei Verwendung von hydrolyseempfindlichen Peroxocarbonsäuren bzw. Carbonsäurehalogeniden oder Carbonsäureanhydriden das Arbeiten in einem 2-Phasen-System empfehlenswert.

In einer besonders bevorzugten Arbeitsweise wird das Pyrazol in einem etwa 9fach molaren Überschuß an einer etwa 50 gew.-%igen wäßrigen Kalilauge gelöst, die Lösung auf etwa 0°C abgekühlt, danach langsam mit etwas mehr als äquimolaren Mengen einer konzentrierten wäßrigen H₂O₂-Lösung versetzt und anschließend portionsweise das Carbonsäurehalogenid oder -anhydrid zugefügt und das Reaktionsgemisch auf ca. 20°C erwärmt.

Im allgemeinen empfiehlt es sich, das Reaktionsgemisch noch einige Zeit bei Raumtemperatur unter Rühren nachreagieren zu lassen und zum Abschluß noch kurz auf etwa 80°C zu erhitzen um noch etwa vorhandenes überschüssiges H₂O₂ bzw. Peroxocarbonsäure zu zersetzen.

Die Aufarbeitung des Reaktionsgemisches erfolgt in an sich bekannter Weise. Beispielsweise säuert man das abgekühlte Reaktionsgemisch mit einer Säure, wie Schwefelsäure an und extrahiert das Reaktionsprodukt mit einem geeigneten Lösungsmittel, beispielsweise Essigsäureethylester. Nach dem Entfernen des Lösungsmittels erhält man ein öl, aus dem das gewünschte N-Hydroxypyrazol kristallisiert. Zur Reinigung kann dieses beispielsweise aus Cyclohexan kristallisiert werden.

Mit Hilfe des erfindungsgemäßen Verfahrens können die als wertvolle Zwischenprodukte für zahlreiche Wirkstoffe begehrten N-Hydroxypyrazole der Formel I auf sehr einfache und vorteilhafte Weise in relativ guten Ausbeuten hergestellt werden.

Die folgenden Beispiele sollen das erfindungsgemäße Verfahren erläutern.

### Beispiel 1

0,4 Mol Pyrazol wurden in 192 g (1.5 Mol) einer 50 gew.%igen wäßrigen KOH gelöst. Die Lösung wurde unter Rühren auf 0°C abgekühlt und langsam mit 34 g (0,5 Mol) einer 50 gew%igen wäßrigen Wasserstoffperoxid-Lösung versetzt. Danach wurden portionsweise jeweils 0,5 Mol des in der folgenden Tabelle angegebenen Carbonsäureanhydrids hinzugefügt und anschließend die Reaktionsmischung auf 20°C erwärmt. Man rührte noch mehrere Stunden, erwärmte zur Zersetzung des Peroxids noch kurz auf 80°C und kühlte das Reaktionsgemisch wieder auf RT ab. Danach säuerte man mit Schwefelsäure an, filtrierte das ausgefallene Kaliumsulfat ab und extrahierte 3 mal mit Essigsäureethylester. Die organischen Phasen wurden getrocknet und das Lösungsmittel entfernt. Man erhielt ein schwach gelbes Öl, aus dem N-Hydroxypyrazol kristallisierte. Das erhaltene N-Hydroxypyrazol wurde aus Cyclohexan kristallisiert. Folgende Versuche wurden durchgeführt:

| Carbonsäureanhydrid | Pyrazol | | | Ausbeute [g/% der Theorie] |
|---|---|---|---|---|
| | R¹ | R² | R³ | |
| Bernsteinsäureanhydrid | H | H | H | 6.7 g/20 % |
| Phthalsäureanhydrid | H | H | H | 23.4 g/70 % |
| Phthalsäureanhydrid | H | Cl | H | 32 g/68 % |

### Beispiel 2

35,3 g (0.5 Mol) Pyrazol wurden in 250 ml Toluol und 73 g (0,65 Mol) einer 50 gew.%igen Kalilauge gelöst. Nach Zugabe von 0,25 g Benzyltriethylammoniumchlorid wurde die Mischung auf 0°C gekühlt. Dann wurden langsam 11,33 g (0,166 Mol) einer 50 %igen wäßrigen Wasserstoffperoxidlösung und danach 23,4 g (0,166 Mol) Benzoylchlorid zugetropft. Anschließend ließ man das Reaktionsgemisch langsam auf 20°C erwärmen und rührte noch mehrere h nach. Zur Aufarbeitung wurde die wäßrige Phase abgetrennt und mit Schwefelsäure angesäuert, wobei die Benzoesäure ausfiel. Nach dem Abfiltrieren des Niederschlags wurde die wäßrige Phase mit Essigsäureethylester ausgeschüttelt, die organische Phase abgetrennt und getrocknet.

Nach Entfernen des Lösungsmittels erhielt man ein Öl, aus dem sich 6,0 g (71,4 mMol) N-Hydroxypyrazol abtrennen ließen.
Ausbeute: 43 % der Theorie, bezogen auf eingesetztes H₂O₂.

Bei Verwendung von Propionsäureanhydrid anstelle von Benzoylchlorid betrug die Ausbeute 23 % der Theorie.

### Beispiel 3

5,1 g (0,075 Mol) Pyrazol wurden in einer Mischung aus 25 ml Wasser und 25 ml Aceton gelöst. Bei Raumtemperatur (RT) wurden 23,6 g (0,075 Mol m-Chlor-perbenzoesäure (55 %ig) zugegeben und die Mischung gerührt. Nach 3 Tagen wurde analog Beispiel 1 aufgearbeitet.
Ausbeute: 2,1 g N-Hydroxypyrazol, entsprechend 30 % der Theorie.

### Beispiel 4

25,65 g (0,3 Mol )4-Chlor-pyrazol wurden in 300 g Wasser suspendiert und anschließend 89,6 g (0,8 Mol) einer 50 %igen wäßrigen KOH-Lösung zugegeben. Nach Rühren bei RT wurden 74,4 g (0,15 Mol) Magnesium-bis-(2-carboxylato-monoperoxibenzoesäure)-hexahydrat (Peroxid-Chemie GmbH) in kleinen Portionen zugefügt. Nach zweitägigem Rühren bei RT wurde analog Beispiel 1 aufgearbeitet.
Ausbeute: 14,4 g (0,12 Mol) 4-Chlor-N-hydroxypyrazol, entsprechend 40 % der Theorie.

### Beispiel 5

7,65 g (0,075 Mol) 4-Chlor-pyrazol wurden in 100 ml Wasser suspendiert und die erhaltene Suspension zunächst mit 72 g (0,64 Mol) einer 50 %igen wäßrigen KOH-Lösung und anschließend bei 0°C mit 17 g (0,15 Mol) einer 30 %igen wäßrigen Wasserstoffperoxidlösung versetzt.

Anschließend wurde das Reaktionsgemisch 5 Minuten gerührt, danach bei 10°C mit 28 g (0,18 Mol) Phthalsäureanhydrid in kleinen Portionen versetzt, dann über Nacht gerührt und anschließend analog Beispiel 1 aufgearbeitet.
Ausbeute: 7,1 g 4-Chlor-N-hydroxypyrazol, entsprechend 80 % der Theorie.

## Patentansprüche

1. Verfahren zur Herstellung von N-Hydroxypyrazolen der allgemeinen Formel I 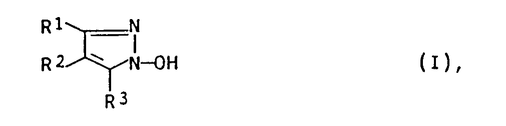 in der
R¹, R² und R³ gleich oder verschieden sein können und Wasserstoff oder Halogen bedeuten können,
dadurch gekennzeichnet, daß man
Pyrazole der allgemeinen Formel II 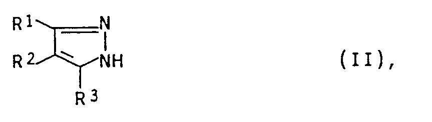 in der
die Reste R¹, R² und R³ die in Formel I angegebene Bedeutung haben, in Gegenwart von 1 bis 15 Mol eines Alkali- oder Erdalkalihydroxids oder -carbonats mit einer aliphatischen oder aromatischen Peroxocarbonsäure in einem stöchiometrischen Verhältnis von Pyrazol II und Peroxocarbonsäure = 1:1 bis 1:2 so umsetzt, daß die Reaktionstemperatur zwischen -5°C und 60°C liegt.

2. Verfahren zur Herstellung von N-Hydroxypyrazolen der allgemeinen Formel I gemäß Anspruch 1, dadurch gekennzeichnet, daß man die Umsetzung in Gegenwart von 1,5 bis 9 Mol eines Alkali- oder Erdalkalihydroxids oder -carbonats durchführt.

3. Verfahren zur Herstellung von N-Hydroxypyrazolen der allgemeinen Formel I gemäß Anspruch 1, dadurch gekennzeichnet, daß man die Pyrazole der allgemeinen Formel II vor der Umsetzung mit der Peroxocarbonsäure in an sich bekannter Weise mit einem Alkalihydroxid, Alkalihydrid oder Alkalicarbonat in ihre Metallsalze der allgemeinen Formel III 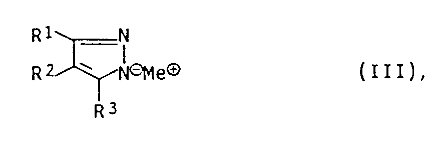 in der
die Reste R¹, R² und R³ die in Formel I genannte Bedeutung haben, und
Me^{⊕} ein Kation der Alkalimetalle bedeutet,
überführt.

4. Verfahren zur Herstellung von N-Hydroxypyrazolen der allgemeinen Formel I gemäß Anspruch 1, dadurch gekennzeichnet, daß die Peroxocarbonsäure in dem Reaktionsgemisch in an sich bekannter Weise aus Wasserstoffperoxid und einem Carbonsäurehalogenid oder Carbonsäureanhydrid hergestellt wird.

5. Verfahren zur Herstellung von N-Hydroxypyrazolen der allgemeinen Formel I gemäß Anspruch 1, dadurch gekennzeichnet, daß man als aromatische Peroxocarbonsäure m-Chlor-perbenzoesäure, Perbenzoesäure oder Monophthalsäure verwendet.

6. Verfahren zur Herstellung von N-Hydroxypyrazolen der allgemeinen Formel I gemäß Anspruch 1, dadurch gekennzeichnet, daß man als aliphatische Peroxocarbonsäure, Peressigsäure, Perpropionsäure, Trifluorperessigsäure oder Monoperbernsteinsäure verwendet.

7. Verfahren zur Herstellung von N-Hydroxypyrazolen der allgemeinen Formel I gemäß Anspruch 1, dadurch gekennzeichnet, daß man die Umsetzung in Wasser als Lösungsmittel durchführt.

8. Verfahren zur Herstellung von H-Hydroxypyrazolen der allgemeinen Formel I gemäß Anspruch 1, dadurch gekennzeichnet, daß man die Umsetzung in einem 2-Phasen-System, bestehend aus Wasser und einem mit Wasser nicht oder nur schlecht mischbaren inerten organischen Lösungsmittel in Gegenwart eines Phasentransferkatalysators durchführt.

9. Verfahren zur Herstellung von H-Hydroxypyrazolen der allgemeinen Formel I gemäß Anspruch 8, dadurch gekennzeichnet, daß man als Phasentransferkatalysator Tetraalkylammoniumsalze der allgemeinen Formel IV 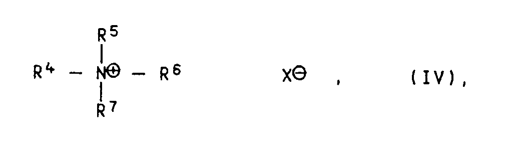 in der
R⁴, R⁵, R⁶ und R⁷ untereinander gleich oder verschieden sein können und für Alkylreste mit 1 bis 22 Kohlenstoffatomen oder Hydroxyl-, Carbonamid- oder Ethergruppen enthaltende Alkylreste mit bis zu 25 Kohlenstoffatomen sowie für Phenylreste oder phenylsubstituierte Alkylreste (wie z.B. Benzyl-) mit bis zu 20 Kohlenstoffatomen stehen können,
und X^{⊖} für ein Anion einer Säure wie J⁻, Cl⁻, Br⁻, (HSO₄)⁻, (CN)⁻, (BF₄)⁻ oder OH steht; verwendet.

10. Verfahren zur Herstellung von N-Hydroxypyrazolen der allgemeinen Formel I gemäß Anspruch 1, dadurch gekennzeichnet, daß man anstelle einer Peroxocarbonsäure ein Alkalimetallsalz oder Erdalkalimetallsalz einer Peroxocarbonsäure verwendet.

11. Verfahren zur Herstellung von N-Hydroxypyrazolen der allgemeinen Formel I gemäß Anspruch 8, dadurch gekennzeichnet, daß man als Phasentransferkatalysator Tetraalkylammoniumsalze der allgemeinen Formel IV 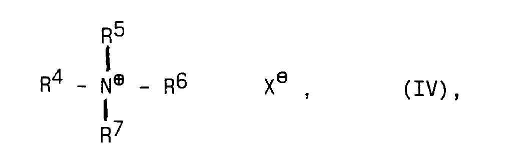 in der
R⁴, R⁵, R⁶ und R⁷ untereinander gleich oder verschieden sein können und für eine Methyl-, Ethyl-, (Iso)propyl-, Butyl-, Octyl-, Dodecyl-, c₁₆H₃₃-, Hydroxy(iso)-propyl- oder 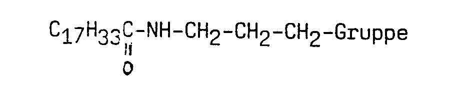 stehen können, und X^{⊖} für ein Anion einer Säure J⁻, Cl⁻, Br⁻, (HSO₄)⁻, (CN)⁻, (BF₄)⁻ oder OH steht,
verwendet.

## Claims

1. A process for the preparation of an N-hydroxypyrazole of the formula I 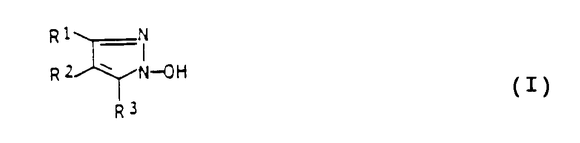 where R¹, R² and R³ may be identical or different and are each hydrogen or halogen, wherein a pyrazole of the formula II 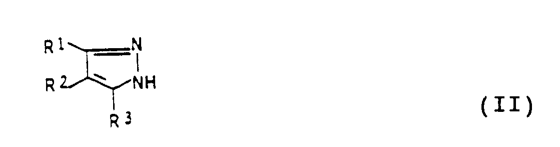 where R¹, R² and R³ have the meanings stated for formula I, is reacted, in the presence of from 1 to 15 mol of an alkali metal hydroxide, alkaline earth metal hydroxide, alkali metal carbonate or alkaline earth metal carbonate, with an aliphatic or aromatic peroxocarboxylic acid in a stoichiometric ratio of pyrazole II and peroxocarboxylic acid of from 1:1 to 1:2 in such a way that the reaction temperature is from -5°C to 60°C.

2. A process for the preparation of an N-hydroxypyrazole of the formula I as claimed in claim 1, wherein the reaction is carried out in the presence of from 1.5 to 9 mol of an alkali metal hydroxide, alkaline earth metal hydroxide, alkali metal carbonate or alkaline earth metal carbonate.

3. A process for the preparation of an N-hydroxypyrazole of the formula I as claimed in claim 1, wherein, before being reacted with the peroxocarboxylic acid, the pyrazole of the formula II is converted in a conventional manner with an alkali metal hydroxide, alkali metal hydride or alkali metal carbonate into one of its metal salts of the formula III 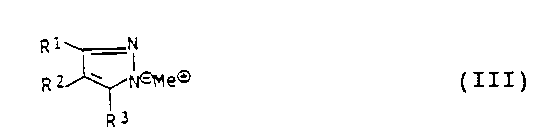 where R¹, R² and R³ have the meanings stated for formula I and Me^{⊕} is a cation of an alkali metal.

4. A process for the preparation of an N-hydroxypyrazole of the formula I as claimed in claim 1, wherein the peroxocarboxylic acid is prepared in a conventional manner in the reaction mixture from hydrogen peroxide and an acyl halide or carboxylic anhydride.

5. A process for the preparation of an N-hydroxypyrazole of the formula I as claimed in claim 1, wherein the aromatic peroxocarboxylic acid used is m-chloroperbenzoic acid, perbenzoic acid or monophthalic acid.

6. A process for the preparation of an N-hydroxypyrazole of the formula I as claimed in claim 1, wherein the aliphatic peroxocarboxylic acid used is peracetic acid, perpropionic acid, trifluoroperacetic acid or monopersuccinic acid.

7. A process for the preparation of an N-hydroxypyrazole of the formula I as claimed in claim 1, wherein the reaction is carried out in water as the solvent.

8. A process for the preparation of an N-hydroxypyrazole of the formula I as claimed in claim 1, wherein the reaction is carried out in a 2-phase system consisting of water and an inert organic solvent which is water-immiscible or poorly miscible with water, in the presence of a phase transfer catalyst.

9. A process for the preparation of an N-hydroxypyrazole of the formula I as claimed in claim 8, wherein the phase transfer catalyst used is a tetraalkylammonium salt of the formula IV 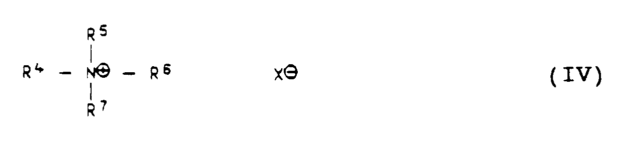 where R⁴, R⁵, R⁶ and R⁷ may be identical or different and are each alkyl of 1 to 22 carbon atoms or alkyl of not more than 25 carbon atoms which contains hydroxyl, carboxamide or ether groups, or are each phenyl or phenyl-substituted alkyl (eg. benzyl) of not more than 20 carbon atoms and X^{⊖} is an anion of an acid, such as I⁻, Cl⁻, Br⁻, (HSO₄)⁻, (CN)⁻, (BF₄)⁻ or OH⁻.

10. A process for the preparation of an N-hydroxypyrazole of the formula I as claimed in claim 1, wherein an alkali metal salt or alkaline earth metal salt of a peroxocarboxylic acid is used instead of a peroxocarboxylic acid.

11. A process for the preparation of an N-hydroxypyrazole of the formula I as claimed in claim 8, wherein the phase transfer catalyst used is a tetraalkylammonium salt of the formula IV 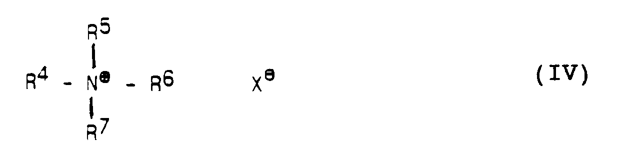 where R⁴, R⁵, R⁶ and R⁷ may be identical or different and are each methyl, ethyl, propyl, isopropyl, butyl, octyl, dodecyl, C₁₆H₃₃, hydroxy(iso)propyl or 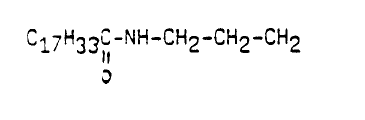 and X^{⊖} is an anion of an acid, such as I⁻, Cl⁻, Br⁻, (HSO₄)⁻, (CN)⁻, (BF₄)⁻ or OH⁻.

## Revendications

1. Procédé de préparation de N-hydroxypyrazoles de formule générale I 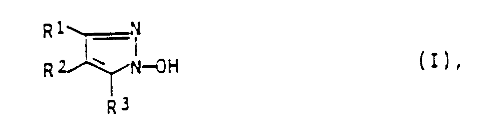 dans laquelle
R¹, R² et R³ peuvent être identiques ou différents et peuvent représenter des atomes d'hydrogène ou d'halogène,
caractérisé en ce qu'on fait réagir des pyrazoles de formule générale II 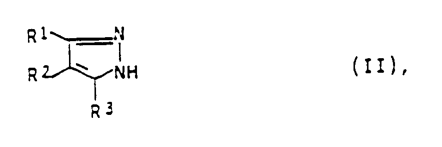 dans laquelle
les restes R¹, R² et R³ ont la signification donnée à propos de la formule I, en présence de 1 à 15 moles d'un hydroxyde ou carbonate de métal alcalin ou alcalinoterreux, avec un acide peroxocarboxylique aliphatique ou aromatique, dans un rapport stoechiométrique du pyrazole II et de l'acide peroxocarboxylique de 1:1 à 1:2, de telle manière que la température de réaction se situe entre -5°C et 60°C.

2. Procédé de préparation de N-hydroxypyrazoles de formule générale I selon la revendication 1, caractérisé en ce qu'on conduit la réaction en présence de 1,5 à 9 moles d'un hydroxyde ou d'un carbonate de métal alcalin ou alcalino-terreux.

3. Procédé de préparation de N-hydroxypyrazoles de formule générale I selon la revendication 1, caractérisé en ce qu'avant la réaction avec l'acide peroxocarboxylique, on transforme de façon connue en soi les pyrazoles de formule générale II, par un hydroxyde de métal alcalin, un hydrure de métal alcalin ou un carbonate de métal alcalin, en leurs sels métalliques de formule générale III 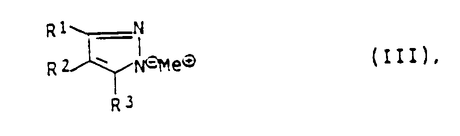 dans laquelle
les restes R¹, R² et R³ ont la signification donnée à propos de la formule I et
M^{⊕} représente un cation des métaux alcalins.

4. Procédé de préparation de N-hydroxypyrazoles de formule générale I selon la revendication 1, caractérisé en ce que l'acide peroxocarboxylique est préparé dans le mélange réactionnel de façon connue en soi à partir de peroxyde d'hydrogène et d'un halogénure d'acide carboxylique ou d'un anhydride carboxylique.

5. Procédé de préparation de N-hydroxypyrazoles de formule générale I selon la revendication 1, caractérisé en ce qu'on utilise, comme acide peroxocarboxylique aromatique, de l'acide m-chloroperbenzoïque, de l'acide perbenzoïque ou de l'acide monophtalique.

6. Procédé de préparation de N-hydroxypyrazoles de formule générale I selon la revendication 1, caractérisé en ce qu'on utilise, comme acide peroxocarboxylique aiiphatique, de l'acide peracétique, de l'acide perpropionique, de l'acide trifluoroperacétique ou de l'acide monopersuccinique.

7. Procédé de préparation de N-hydroxypyrazoles de formule générale I selon la revendication 1, caractérisé en ce qu'on conduit la réaction dans de l'eau servant de solvant.

8. Procédé de préparation de N-hydroxypyrazoles de formule générale I selon la revendication 1, caractérisé en ce qu'on conduit la réaction dans un système à 2 phases, se composant d'eau et d'un solvant organique inerte qui n'est que peu, sinon pas miscible à l'eau, en présence d'un catalyseur de transfert de phase.

9. Procédé de préparation de N-hydroxypyrazoles de formule générale I selon la revendication 8, caractérisé en ce qu'on utilise, comme catalyseur de transfert de phase, des sels de tétraalkylammonium de formule générale IV 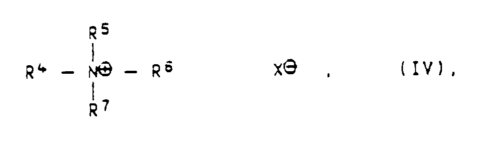 dans laquelle
R⁴, R⁵, R⁶ et R⁷ peuvent être identiques ou différents les uns des autres et peuvent être mis pour des restes alkyle à 1-22 atomes de carbone, pour des restes alkyle contenant des groupements hydroxy, carboxamide ou éther et renfermant Jusqu'à 25 atomes de carbone, ou pour des restes phényle ou des restes alkyle phénylsubstitués (par exemple benzylsubstitués) renfermant jusqu'à 20 atomes de carbone,
et X^{⊖} est mis pour un anion d'un acide, tel que I⁻, Cl⁻, Br-, (HSO₄)⁻, (CN)⁻, (BF₄)⁻ ou OH.

10. Procédé de préparation de N-hydroxypyrazoles de formule générale I selon la revendication 1, caractérisé en ce qu'on utilise, à la place d'un acide peroxocarboxylique, un sel de métal alcalin ou un sel de métal alcalino-terreux et d'acide peroxocarboxylique.

11. Procédé de préparation de N-hydroxypyrazoles de formule générale I selon la revendication 8, caractérisé en ce qu'on utilise, comme catalyseur de transfert de phase, des sels de tétraalkylammonium de formule générale IV 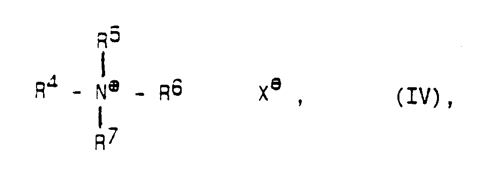 dans laquelle
R⁴, R⁵, R⁶ et R⁷ peuvent être identiques ou différents les uns des autres et peuvent être mis chacun pour un groupement méthyle, éthyle, (iso)propyle, butyle, octyle, dodécyle, C₁₆H₃₃, hydroxy(iso)propyle ou 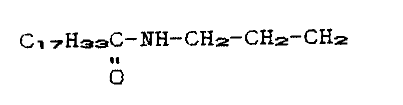 et X⁻ est mis pour un anion d'un acide, tel que I⁻, Cl⁻, Br⁻, (HSO₄)⁻, (CN)⁻, (BF₄)⁻ ou OH.
